(19) Europäisches Patentamt / European Patent Office / Office européen des brevets

(11) **EP 4 180 801 B1**

(12) **EUROPÄISCHE PATENTSCHRIFT**

(45) Veröffentlichungstag und Bekanntmachung des Hinweises auf die Patenterteilung:
**10.01.2024   Patentblatt 2024/02**

(51) Internationale Patentklassifikation (IPC):
**G01N 27/02** *(2006.01)*      *C21B 13/00* *(2006.01)*

(21) Anmeldenummer: **21208339.8**

(52) Gemeinsame Patentklassifikation (CPC):
**G01N 27/023; C21B 7/24; C21B 13/00; C21B 13/0086;** C21B 2300/04

(22) Anmeldetag: **15.11.2021**

(54) **VERFAHREN ZUR GEHALTSBESTIMMUNG ZUMINDEST VON METALLISCHEM EISEN IN DURCH DIREKTREDUKTION AUS EISENERZ HERGESTELLTEM EISENSCHWAMM ODER EINER PROBE DAVON**

METHOD FOR DETERMINING THE CONTENT OF AT LEAST METALLIC IRON IN SPONGE IRON PRODUCED BY DIRECT REDUCTION FROM IRON ORE OR A SAMPLE THEREOF

PROCÉDÉ DE DÉTERMINATION D'AU MOINS LA TENEUR EN FER MÉTALLIQUE DANS UNE ÉPONGE DE FER OU DANS UN ÉCHANTILLON DE CELLE-CI FABRIQUÉ PAR RÉDUCTION DIRECTE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**17.05.2023   Patentblatt 2023/20**

(73) Patentinhaber: **voestalpine Stahl GmbH**
**4020 Linz (AT)**

(72) Erfinder:
- **Feilmayr, Christoph**
  **4020 Linz (AT)**
- **Gangl, Erfried**
  **4492 Hofkirchen (AT)**
- **Thaler, Christoph**
  **4020 Linz (AT)**
- **Pichler, Anton**
  **3311 Zeillern (AT)**
- **Griesser, Anna Sonja**
  **4560 Kirchdorf an der Krems (AT)**

- **Mauhart, Joachim**
  **4595 Waldneukirchen (AT)**
- **Schuster Stefan**
  **4470 Enns (AT)**
- **Scheiblhofer, Stefan**
  **4470 Enns (AT)**
- **Gstöttenbauer, Norbert**
  **4209 Engerwitzdorf (AT)**
- **Harris, Christopher**
  **4020 Linz (AT)**
- **Amrhein, Wolfgang**
  **4040 Linz (AT)**
- **Wöckinger, Daniel**
  **4040 Linz (AT)**

(74) Vertreter: **Jell, Friedrich**
**Bismarckstrasse 9**
**4020 Linz (AT)**

(56) Entgegenhaltungen:
**EP-A1- 3 757 233      DE-A1- 3 017 001**
**JP-A- 2002 088 417      JP-B2- 5 936 006**

**Beschreibung**

Die Erfindung betrifft ein Verfahren zur Gehaltsbestimmung zumindest von metallischem Eisen in durch Direktreduktion aus Eisenerz hergestelltem Eisenschwamm.

Zur Bestimmung des Metallisierungsgrads von Eisenschwamm oder einer Probe davon, nämlich eines Messvolumens des Eisenschwamms, ist es aus dem Stand der Technik bekannt (DE3017001A1), eine Messgröße, nämlich die Impedanz einer Messspule, zu erfassen, die mit einer Erregerspule über den Eisenschwamm oder einer Probe davon gekoppelt ist. Die Erregerspule prägt zeitlich ändernde magnetische Felder mit voneinander unterschiedlichen Frequenzen in den Eisenschwamm oder einer Probe davon ein. Damit ist die Messgröße von mindestens einer elektromagnetischen Eigenschaft des Eisenschwamms oder des Teils davon abhängig. Nach der DE3017001A1 soll die Impedanz der Messspule eine gute Beziehung zur Volumenleitfähigkeit des Eisenschwamms oder einer Probe davon und damit zu dessen Metallisierungsgrad aufweisen. Nachteilig bei diesem Verfahren ist die vergleichsweise hohe Ungenauigkeit in der Beziehung zwischen Messdaten und Volumenleitfähigkeit, was lediglich eine grobe Abschätzung des Metallisierungsgrads erlaubt. Folglich ist ein derartiges Verfahren auch beispielsweise für eine exakte Regelung eines Direktreduktionsverfahrens nicht geeignet.

Die Erfindung hat sich daher die Aufgabe gestellt, ein Verfahren zur Gehaltsbestimmung zumindest von metallischem Eisen in Eisenschwamm in seiner Genauigkeit zu verbessern.

Die Erfindung löst die gestellte Aufgabe durch die Merkmale des Anspruchs 1.

Die Genauigkeit des Verfahrens kann deutlich erhöht werden, wenn nicht nur -wie im Stand der Technik geoffenbart- eine Beziehung zwischen einer erfassten elektrischen Messgröße und einer elektromagnetischen Eigenschaft des Eisenschwamms verwendet wird, um den Gehalt von mindestens einem Element des Eisenschwamms oder einer Probe davon zu bestimmen, sondern wenn hierzu ein mathematisches Modell verwendet wird. Insbesondere erfindungsgemäß dadurch, dass ein mathematisches Modell bereitgestellt wird, dessen Modellausgangsgröße als Funktion des Gehalts zumindest von metallischem Eisen ($Fe_{met}$) des Eisenschwamms oder einer Probe davon beschrieben wird, wobei dieses mathematische Modell eine Effektive-Medium-Approximation (EMA) für die Permeabilität ($\mu_{eff}$) und für die elektrische Leitfähigkeit ($\sigma_{eff}$) des Eisenschwamms oder einer Probe davon aufweist. Es hat sich nämlich überraschend herausgestellt, dass mit einer Effektive-Medium-Approximation (EMA) für die Permeabilität und für die elektrische Leitfähigkeit die Gehaltsbestimmung deutlich genauer und/oder schneller erfolgen kann, wenn ein Schätzverfahren zur Gehaltsbestimmung zumindest von metallischem Eisen ($Fe_{met}$) unter Verwendung der erfassten Messgröße und des mathematischen Modells durchgeführt wird. Das erfindungsgemäße schnelle und genaue Verfahren eignet sich daher auch besonders für eine Überwachung, Regelung oder Steuerung eines Verfahrens zur Direktreduktion von Eisenerz.

Vorzugsweise geht bzw. gehen in das mathematische Modell ein Gangartanteil ($p_g$) des Eisenerzes oder Eisenschwamms oder einer Probe davon als erster Eingangsparameter und/oder eine, insbesondere effektive, Dichte (ptot) des Eisenschwamms oder einer Probe davon als zweiter Eingangsparameter ein.

Wird der Gangartanteil als erster Eingangsparameter vorgegeben, können Ungenauigkeiten in der Gehaltsbestimmung weiter verringert werden, da durch diese Maßnahme die eisenhaltigen Anteile (metallisches Eisen, Eisenoxide, Eisenkarbid) im Feststoffanteil des Messvolumens weitaus genauer bestimmt werden können. Vorzugsweise kann der Gangartanteil ein veränderbarer Eingangsparameter sein, der sich aus dem verwendeten Eisenerz ergibt, und der in das mathematische Modell eingesetzt werden kann. Dies kann die Genauigkeit des Verfahrens weiter erhöhen. Die Dichte des Eisenschwamms oder einer Probe davon als zweiter Eingangsparameter kann beispielsweise durch Wiegen oder durch die Bestimmung aus dem verwendeten Eisenerz verfahrenstechnisch einfach bestimmt werden. Damit kann beispielsweise der gasförmige Anteil exakt bestimmt werden, und -da sowohl die magnetischen als auch die elektrischen Parameter des Gasanteils bekannt sind- kann die Gehaltsbestimmung besonders genau und robust gegenüber Störeinflüssen durchgeführt werden. Vorzugsweise kann diese Dichte ein veränderbarer Eingangsparameter sein, der sich aus dem verwendeten Eisenerz ergibt, und der in das mathematische Modell eingesetzt werden kann. Dies kann die Genauigkeit des Verfahrens weiter erhöhen.

Ist das mathematische Modell ein elektromagnetisches Modell (EMM) kann die Genauigkeit des Verfahrens weiter erhöht werden. Insbesondere, weil damit die modellierte Größe die elektrische Messgröße genauer abbilden kann.

Ist die Modellausgangsgröße $\underline{A}_{mod}$ eine elektrische Impedanz $\underline{Z}_{mod}(\omega_i)$ ist, können die Anteile magnetischer und unmagnetischer Stoffe/Materialien wie metallischem Eisen $Fe_{met}$, Eisenoxid FeO sowie Eisenkarbid $Fe_3C$ verbessert bestimmt werden.

Vorzugsweise weist das Impedanzmodell eine Modellierung zumindest einer elektrischen Spule mit Eisenschwamm oder einer Probe davon als Material des Kerns aufweist.

Wird eine elektrische Spannung $\underline{U}_{mod}(\omega_i)$ modelliert, die sich unter Berücksichtigung eines bekannten, insbesondere eingeprägten, Stroms an der elektrischen Impedanz des Impedanzmodells bzw. modellierten Impedanz bildet, können die Modell-Parameter der Anteile magnetischer und unmagnetischer Stoffe wie Eisen und/oder Eisenoxid und/oder Eisenkarbid bestimmt werden, was die Genauigkeit des Verfahrens weiter erhöhen kann.

Alternativ ist vorstellbar, dass ein elektrischer Strom $\underline{I}_{mod}(\omega_i)$ modelliert wird, der sich beim Anlegen einer bekannten, insbesondere eingeprägten, Spannung an die elektrischen Impedanz des Impedanzmodells bzw. modellierten Impedanz einstellt.

Im Gegensatz zum Stand der Technik wird eine besonders exakte Gehaltsbestimmung ermöglicht, wenn durch das Schätzverfahren jener Gehalt oder jene Gehälter bestimmt werden, für welche die Abweichung der mindestens einen erfassten Messgröße ($A_{mess}$) zur Modellausgangsgröße ($A_{mod}$) des mathematischen Modells minimal ist. Vorzugsweise werden durch das Schätzverfahren jener Gehalt oder jene Gehälter bestimmt, für welche die Abweichung der Summe der erfassten Messgrößen ($A_{mess}$) zur Modellausgangsgröße ($A_{mod}$) des mathematischen Modells minimal ist.

Mithilfe dieses Schätzverfahrens kann es nämlich ermöglicht werden, ein äußerst sensitives stochastisches Verfahren zu schaffen - was den Aufwand für die Messdatenerfassung vereinfacht. So kann es beispielsweise ausreichen, bereits mit dem Anlegen von zwei magnetischen Feldern, ausreichende Messdaten zu erfassen, um auf den Gehalt an metallischem Eisen und/oder Eisenoxid und/oder Eisenkarbid im Eisenschwamm äußerst exakt schließen zu können. Insbesondere kann sich hierfür ein Least-Squares-Schätzverfahren besonders auszeichnen.

Vorzugsweise wird als oder für die Messgröße $A_{mess}$ jene in einer Spule induzierte elektrische Spannung $\underline{U}_{mess}$ erfasst wird, die sich aus dem magnetischen Feld ergibt, das durch einen eingeprägten Strom $\underline{I}$ erzeugt wird, wobei die Spule Eisenschwamm oder eine Probe davon als Material des Kerns aufweist.

Alternativ ist vorstellbar, dass als oder für die Messgröße $A_{mess}$ jener elektrische Strom $I_{mess}$ erfasst wird, der sich in der Spule einstellt, wenn an dieser Spule eine eingeprägte Spannung U angelegt wird, wobei die Spule Eisenschwamm oder eine Probe davon als Material des Kerns aufweist.

Die Genauigkeit des Verfahrens kann weiter verbessert werden, wenn erste Messdaten der Messgröße durch Induzieren eines ersten magnetischen Felds in den Eisenschwamm oder einer Probe davon und zweite Messdaten der Messgröße durch Induzieren eines zweiten magnetischen Felds in den Eisenschwamm oder einer Probe davon erfasst werden, wobei sich die magnetischen Felder zeitlich ändern und zueinander unterschiedliche Frequenzen aufweisen. Durch die Erfassung weiterer Messdaten bei weiteren Frequenzen kann die Genauigkeit weiter erhöht werden.

Vorzugsweise kann die Modellausgangsgröße ($A_{mod}$) als Funktion des Gehalts zumindest von metallischem Eisen ($Fe_{met}$) sowie von mindestens einem Eisenoxid ($Fe_2O_3$, $Fe_3O_4$ bzw. FeO) als auch von Eisenkarbid ($Fe_3C$) des Eisenschwamms oder einer Probe (4) davon beschrieben werden. Damit kann das Schätzverfahren zur Gehaltsbestimmung zumindest von metallischem Eisen ($Fe_{met}$), Eisenoxid ($Fe_2O_3$, $Fe_3O_4$ bzw. FeO) und Eisenkarbid ($Fe_3C$) unter Verwendung der erfassten Messgröße (Amess) und des mathematischen Modells durchgeführt werden.

In der Figur ist beispielsweise der Erfindungsgegenstand anhand eines Ausführungsbeispiels näher dargestellt. Es zeigen

Fig. 1 zeigt ein mathematisches Modell 1 in vereinfachter Darstellung und
Fig. 2 eine Prinzipdarstellung zu einem Messaufbau.

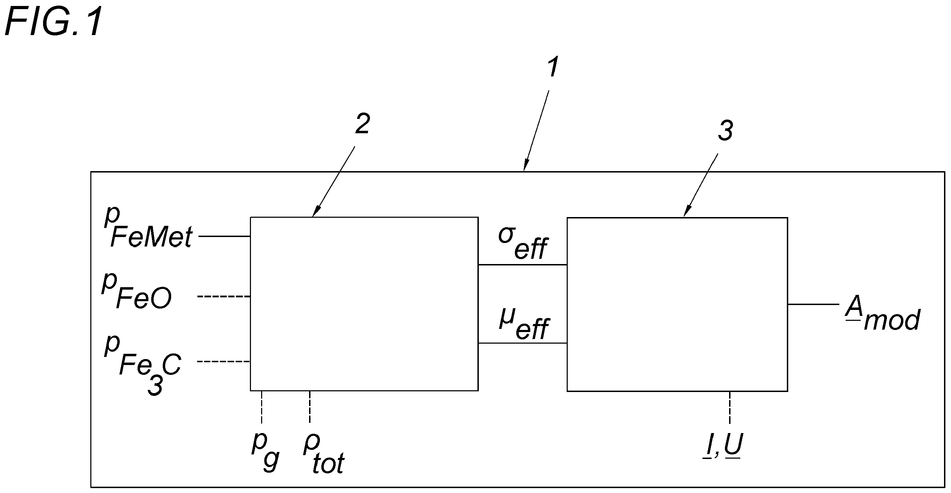

Das nach Fig. 1 dargestellte mathematische Modell 1 wird zusammen mit einer elektrischen Messgröße $\underline{U}_{mess}$ zur Bestimmung des Gehalts $\underline{p}_{FeMet}$ von metallischem Eisen $Fe_{met}$, zur Bestimmung des Gehalts $\underline{p}_{FeO}$ von Eisenoxid FeO und zur Bestimmung des Gehalts $\underline{p}_{Fe3C}$ von Eisenoxid $Fe_3C$ verwendet.

Das mathematische Modell 1 beschreibt die elektromagnetischen Eigenschaften des Eisenschwamms oder einer Probe 4 davon in Abhängigkeit des Gehalts $p_{FeMet}$ an metallischem Eisen $Fe_{met}$, des Gehalts $p_{FeO}$ von Eisenoxid FeO und des Gehalts $p_{Fe3C}$ von Eisenoxid $Fe_3C$. Dabei ist jeweils $p_i$ mit i = {FeMet, Fe0, $Fe_3O_4$, ...} jene auf die Gesamtmasse des Materials im Probenbehälter bezogene Masse, beispielsweise $p_{FeMet}$ die auf die Gesamtmasse des Materials im Probenbehälter bezogene Masse an metallischem Eisen $Fe_{met}$.

Neben dieser sind für die Abschätzung der Qualität von direkt reduzierten Eisen oftmals noch zusätzlich die Massen von Eisenoxiden und Eisenkarbiden von Interesse. Aufgrund der Zusammensetzung des eingesetzten Rohmaterials befindet sich auch Gangart im direkt reduzierten Material. Zusammengefasst besteht das zu untersuchende Material aus metallischem Eisen $Fe_{met}$, Eisenoxiden und Eisenkarbiden mit einem Massenanteil $p_i$ an der Gesamtmasse und aus zu Gangart zusammengefassten Anteilen an nicht eisenhaltigen Stoffen mit einem Masseanteil $p_g$ an der Gesamtmasse.

Dieser Gangartanteil $p_g$ kann mithilfe verschiedenster Methoden (z. B. chemischen Analysen) bestimmt werden, oder ist der Gangartanteil $p_g$ als typische Kenngröße für Rohmaterial bestimmter Abbaugebiete bekannt, kann die Masse der Gangart bestimmt werden, wodurch die Gesamtmasse von Eisen, Eisenkarbiden und Eisenoxiden innerhalb des Volumens berechnet werden kann. Schließlich muss durch ein mathematisches Modell der Anteil an metallischem Eisen von der verbleibenden Masse abgetrennt werden. Diese geschieht erfindungsgemäß anhand der elektromagnetischen Eigenschaften der unterschiedlichen Inhaltsstoffe.

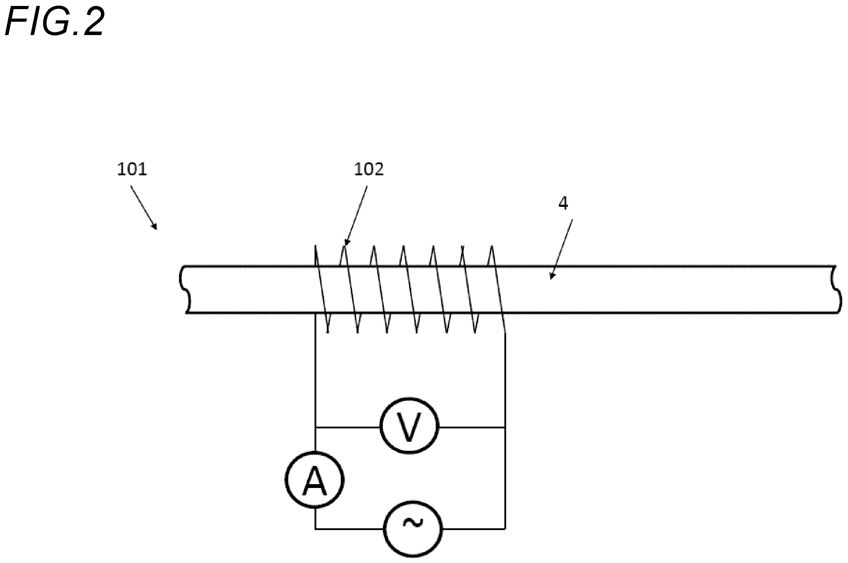

In einer bevorzugten Variante umfasst der nach Fig. 2 dargestellte Messaufbau 100 eine Spule 101 in Form einer Zylinderspule, die den Eisenschwamm bzw. eine Probe 4 davon umschließt und damit als Kernmaterial verwendet. Alternativ zur nach Fig. 2 dargestellten Spule können auch eine oder mehrere Erregerspulen in Kombination mit einer oder mehrere Messpulen verwendet werden, was nicht näher dargestellt worden ist.

Durch diesen Messaufbau ergibt sich ein vereinfachtes Impedanzmodell 3, das sich aus einer analytischen Beschreibung der Impedanz eines homogenen Zylinders ergibt, wobei dessen elektromagnetische Eigenschaften vom Eisengehalt beeinflusst werden.

Der Zusammenhang der Messgröße $\underline{U}_{mess}$ und den magnetischen Eigenschaften der Probe 4 ergibt sich aus der Lösung des magnetischen Kreises 4, d. h. es fließen neben den elektrischen und magnetischen Eigenschaften der Probe 4, die Geometrie des Aufbaus und die elektrischen Eigenschaften des Messaufbaus (wie z. B. der Durchmesser der Zylinderspule 101 und der Widerstand der Spule 101) ein. Diese prinzipielle Vorgehensweise wird weiter erläutert.

Gemäß einem Ausführungsbeispiel wird das Probenmaterial in einen schlanken, zylinderförmigen, nichtmagnetischen, elektrisch nichtleitenden Behälter eingebracht. Um diesen Behälter ist eine kurze ringförmige Spule 101 angeordnet, die sowohl als Erregerspule, als auch als Messspule fungieren kann.

Für die Modellierung eines Impedanzmodells 3 kann für die modellierte Impedanz Z dieses Ausführungsbeispiels einer Messanordnung kann vereinfacht angenommen werden, dass es sich beim Probenvolumen um einen im Verhältnis zur Höhe der Messspule näherungsweise unendlich langen Zylinder und beim Messsystem um eine das Probenvolumen umfassende Zylinderspule handelt (vgl. H. Libby, Introduction to Electromagnetic Nondestructive Test Methods, Wiley, 1971). Damit kann die Impedanz $\underline{Z}$ des Messsystems mithilfe eines mathematischen elektromagnetischen Modells (EMM)

$$\underline{Z} = \omega\mu_{\text{eff}}\pi a^2 \left[ \frac{2}{ka} \frac{\text{ber}'(k*a) + j\,\text{bei}'(k*a)}{\text{ber}(k*a) + j\,\text{bei}(k*a)} \right] + j\omega\mu_{\text{eff}}\pi(b^2 - a^2)$$

mit

| | |
|---|---|
| $\omega$ | Kreisfrequenz des eingeprägten Wechselstroms |
| $\mu_{\text{eff}}$ | effektive Permeabilität des Probenvolumens |
| a | mittlere Radius des Probenvolumens ist |
| b | mittleren Radius der Zylinderspule |
| ber(k*a) | Realteil Kelvin-Bessel-Funktion |
| ber'(k*a) | 1 Ableitung von ber(k*a) |
| bei(k*a) | Imaginärteil der Kelvin-Bessel-Funktion |
| bei'(k*a) | 1 Ableitung von bei(k*a) |
| k | charakteristische Zahl des Wirbelstromproblems |

und

$$k = \sqrt{\omega\mu_{\text{eff}}\sigma_{\text{eff}} + j\omega^2\mu_{\text{eff}}\varepsilon_0}$$

mit

| | |
|---|---|
| $\omega$ | Kreisfrequenz des eingeprägten Wechselstroms |
| $\mu_{\text{eff}}$ | effektive Permeabilität des Probenvolumens |
| $\sigma_{\text{eff}}$ | elektrische Leitfähigkeit des Probenvolumens |
| $\varepsilon_0$ | Elektrische Feldkonstante (8,854 $10^{-12}$ As/Vm) |

modelliert werden.

Um das mathematische Modell 1 für die Gehaltsbestimmung zu vervollständigen und damit für die Materialaufteilung zu erhalten, weist das mathematische Modell 1 eine Effektive-Medium-Approximation (EMA) 2 für die effektive Permeabilität $\mu_{\text{eff}}$ und für die effektive elektrische Leitfähigkeit $\sigma_{\text{eff}}$ des Eisenschwamms oder einer Probe 4 davon auf. Die EMA 2 stellen grundsätzlich einen mathematischen Zusammenhang zwischen effektiven Stoffeigenschaften und den einzelnen Inhaltsstoffen und dessen Volumenanteile her.

Der Einfachheit halber wird in diesem Ausführungsbeispiel die Probe 4 als aus zwei Materialphasen bestehend angenommen, einerseits metallisches Eisen $Fe_{met}$, ein ferromagnetisches Material mit einer guten Leitfähigkeit, und den restlichen Bestandteilen andererseits schwach ferromagnetisches Material mit einer sehr schlechten bzw. nicht vorhandenen Leitfähigkeit. Hier wird also angenommen, dass das metallische Eisen $Fe_{met}$ mit einem Volumenanteil $f_{FeMet}$ das ferromagnetische Material mit einer guten Leitfähigkeit darstellt, und Eisenoxide, Gangart und Luft in ihrer Summe das schwach ferromagnetische Material mit einer vergleichsweise schlechten Leitfähigkeit darstellt.

So genügt bei der Bestimmung des auf das Volumen des Probevolumens bezogenen Anteil $f_{FeMet}$ des metallischen Eisens $Fe_{met}$ gemäß dem Ausführungsbeispiel eine "inverse Maxwell-Garnett-Formel" für zwei Materialphasen (vgl. J. C. Maxwell-Garnett, Colours in metal glasses and in metal films, Trans. Royal Soc. London, vol. 203, pp. 385-420, 1904)

$$\mu_{eff} = \mu_{FeMet} + 3(1 - f_{FeMet})\mu_{FeMet} \frac{\mu_{res} - \mu_{FeMet}}{\mu_{res} + 2\mu_{FeMet} - (1 - f_{FeMet})(\mu_{res} - \mu_{FeMet})}$$

mit

$\mu_{FeMet}$   bekannte Permeabilität von reinem Eisen (Im Frequenzbereich bis 100Hz liegt diese für magnetische Flussdichten unter 0,9T bei ca. 5000),

$\mu_{res}$   unbekannte Permeabilität des nicht ferromagnetischen Materials

$\mu_{eff}$   resultierende effektive Permeabilität des Materials

$f_{FeMet}$   gesuchter volumetrischer Anteil an metallischem Eisen $Fe_{met}$

Ähnlich wird auch die effektive elektrische Leitfähigkeit $\sigma_{eff}$ des Materials modelliert. In diesem Fall lautet die inverse Maxwell-Garnett-Formel für zwei Materialphasen

$$\sigma_{eff} = \sigma_{FeMet} + 3(1 - f_{FeMet})\sigma_{FeMet} \frac{\sigma_{res} - \sigma_{FeMet}}{\sigma_{res} + 2\sigma_{FeMet} - (1 - f_{FeMet})(\sigma_{res} - \sigma_{FeMet})}$$

mit

$\sigma_{FeMet}$   bekannte elektrische Leitfähigkeit von reinem Eisen (Im Frequenzbereich bis 100Hz liegt diese bei ca. 10Sm/mm$^2$)

$\sigma_{res}$   elektrische Leitfähigkeit des nicht ferromagnetischen Materials mit einer vergleichsweise schlechten Leitfähigkeit. In einer weiteren Vereinfachung kann diese Leitfähigkeit mit Null angenommen werden.

$\sigma_{eff}$   resultierende effektive elektrische Leitfähigkeit des Materials

$f_{FeMet}$   gesuchter volumetrischer Anteil an metallischem Eisen

Je nach Art des Rohmaterials eigenen sich durch deren Materialmischung, deren räumlichen Verteilung und deren geometrischen Proportionen verschiedenste EMAs für eine Modellierung zwei- und mehrphasiger Materialmischungen. Soll beispielsweise nicht nur der Anteil $p_{FeMet}$ an metallischem Eisen $Fe_{met}$, sondern auch der Anteil anderer metallischer oder elektrisch leitfähiger Materialien, insbesondere Eisenoxiden und/oder Eisencarbiden, bestimmt werden, kann hierzu ein Modellierungsansatz für mehrphasige Materialmischungen gewählt werden (z. B.: A. H. Sihvola and I. V. Lindell, Effective permeability of mixtures, Prog Electromagn. Res. 06, pp. 153-180, 1992).

Typischerweise werden in diesen Formeln nicht die massenbezogenen Gehälter $p_i$ der einzelnen Inhaltsstoffe zur Abschätzung der effektiven elektromagnetischen Größen verwendet, sondern die volumenbezogenen Größen $f_i$. Die Umrechnung kann mithilfe der Materialdichte und der gesamten effektiven Dichte des Probenbehälters $\rho_{tot}$ erfolgen.

$$f_i = \frac{\rho_{tot}}{\rho_i} p_i$$

Im mathematischen Modell 1 können die effektive Dichte ptot und der Gangartanteil $p_g$ der Probe 4 als Eingangsparameter modelliert werden. Dies erfolgt beispielsweise, indem üblicherweise bekannte Werte des eingesetzten Grundmaterials verwendet werden. Es können aber genauso gut der Gangartanteil $p_g$ und die relative Dichte ptot im Probenbehälter exakt bestimmt werden, in dem diese über die vorab bestimmte Masse des Materials der Probe 4 und das bekannte Volumen des Probenbehälters berechnet werden.

Unter Verwendung der EMA Gleichungen für die effektive Permeabilität des Probenvolumens ($\mu_{eff}$) und die elektrische Leitfähigkeit des Probenvolumens ($\sigma_{eff}$), mit zwei Unbekannten, Permeabilität des nicht ferromagnetischen Materials ($\mu_{res}$) und gesuchter volumetrischer Anteil $f_{FeMet}$ an metallischem Eisen $Fe_{met}$ kann der volumetrische Anteil $f_{FeMet}$ an metallischem Eisen $Fe_{met}$ des Eisenschwamms bzw. der Probe 4 bestimmt werden.

Da sich die verschiedenen Modelle, hauptsächlich bei mehrphasigen Materialmischungen, nicht explizit ineinander eingesetzt werden können, kann bei der Lösung des Gesamtmodells auf ein numerisches Schätzverfahren zurückgegriffen werden. Ein mögliches Verfahren ist zum Beispiel ein Least-Squares-Schätzverfahren. Über ein Least-Squares-Schätzverfahren können jene Gehalte an metallischem Eisen (Fe-$_{met}$) bestimmt werden, bei denen die Abweichung der modellierten Größe $A_{mod}$ und der elektrischen Messgröße $A_{mess}$ ein Minimum aufweisen. Dies gilt auch für das nach Fig. 1 optional dargestellte Eisenoxid (FeO) sowie das nach Fig. 1 optional dargestellte Eisenkarbid (Fe$_3$C) der Probe 4 bei einem entsprechenden mathematischen Modell 1, wobei in Fig. 1 optional an der strichlierten Darstellung erkannt werden kann.

Ist also die komplexe Impedanz bekannt, kann die effektive Permeabilität des Materials sowie die effektive elektrische Leitfähigkeit des Materials berechnet und daraus wiederum die volumetrische Zusammensetzung unter Anwendung einer EMA berechnet werden.

Die komplexe Impedanz des Messsystems wiederum kann aus dem gemessenen Strom bei einem Anlegen einer definierten Spannung oder aus der gemessenen Spannung beim Einleiten eines definierten Stroms bestimmt werden.

Für die Ausführung des Messablaufs eignen sich als Mess- ($\underline{A}_{mess}$) und Modellausgangsgröße ($\underline{A}_{mod}$) insbesondere die induzierte Spannung in einer Spule ($\underline{U}_{mess}$, $\underline{U}$-$_{mod}$), die komplexe Impedanz der Messanordnung ($\underline{Z}_{mess}$, $\underline{Z}_{mod}$) oder auch der Strom durch eine Mess- oder Erregerspule ($\underline{I}_{mess}$, $\underline{I}_{mod}$).

In einer bevorzugten Ausführungsform wird eine Impedanz als Modellausgangsgröße $A_{mod}$ modelliert, d. h. $\underline{A}_{mod}$ ist eine Impedanz $\underline{Z}_{mod}$. Dabei sind die Schritte des Verfahrens wie folgt der Reihe nach:

    a. Bereitstellen eines mathematischen Models. Das Modell beschreibt die Impedanz $\underline{Z}_{mod}(\omega)$ einer Probe 4 eines Eisenschwamms in einem Messkreis in Abhängigkeit der elektrischen Frequenz w, des Gehalts an metallischem Eisen (Fe$_{met}$), Eisenoxiden (Fe$_2$O$_3$, Fe$_3$O$_4$ bzw. FeO) und Eisenkarbid (Fe$_3$C) des Eisenschwamms bzw. der Probe 4 davon.

    Für diese Beschreibung weist das mathematische Modell 1 einerseits eine Bestimmung der Impedanz in Abhängigkeit der Permeabilität ($\mu_{eff}$) und der elektrischen Leitfähigkeit ($\sigma_{eff}$) des Eisenschwamms oder einer Probe 4 davon auf. Andererseits weist das Modell eine Bestimmung der Permeabilität ($\mu_{eff}$) und für die elektrische Leitfähigkeit ($\sigma_{eff}$) in Abhängigkeit des Gehalts an metallischem Eisen (Fe$_{met}$), Eisenoxiden (Fe$_2$O$_3$, Fe$_3$O$_4$ bzw. FeO) und Eisenkarbid (Fe$_3$C) des Eisenschwamms, sowie des Gangartanteils und der Dichte auf, wozu eine Effektive-Medium-Approximation (EMA) zur Anwendung kommt.

    b. Einsetzen der effektive Dichte ptot der Probe 4 und des Gangartanteils $p_g$ der Probe 4 in das mathematische Modell 1.

    c. Einbringen einer Probe 4 des Eisenschwamms in den magnetischen Kreis der Messanordnung.

    d. Einprägen eines ersten Stromverlaufs $\underline{I}(\omega_1)$ in die Erregerspule samt Bestimmung/Erfassung der Impedanz $\underline{Z}_{mess}(\omega_1)$ als Messgröße ($A_{mess}$) aus der Messung eines ersten Spannungsverlaufs $\underline{U}_{mess}(\omega_1)$ an der Messspule.

    e. Einprägen eines zweiten Stromverlaufs $\underline{I}(\omega_2)$ in die Erregerspule samt Bestimmung/Erfassung der Impedanz $\underline{Z}_{mess}(\omega_2)$ als Messgröße ($A_{mess}$) aus der Messung eines zweiten Spannungsverlaufs $\underline{U}_{mess}(\omega_2)$ an der Messspule.

    f. Optional: Einprägen weiterer Stromverläufe $\underline{I}(\omega_i)$ in die Erregerspule samt Bestimmung/Erfassung der Impedanzen $\underline{Z}_{mess}(\omega i)$ als Messgröße ($A_{mess}$) aus den Messungen weiterer Spannungsverläufe $\underline{U}_{mess}(\omega_i)$ an der Messspule.

    g. Bestimmung der Anteile an metallischem Eisen (Fe$_{met}$) und/oder Eisenoxid (Fe$_2$O$_3$, Fe$_3$O$_4$ bzw. FeO) und/oder Eisenkarbid (FesC) in der Probe 4 an Eisenschwamm durch Anwendung eines Least-Squares-Schätzverfahrens unter Verwendung des mathematischen Modells 1 und der Messdaten für die Spannungsverläufe $\underline{U}_{mess}(\omega_1)$, $\underline{U}_{mess}(\omega_2)$ und optional $\underline{U}_{mess}(\omega_i)$.

Dabei werden jene Anteile an metallischem Eisen (Fe$_{met}$) und/oder Eisenoxid (Fe$_2$O$_3$, Fe$_3$O$_4$ und FeO) und/oder Eisenkarbid (Fe$_3$C) in der Probe 4 ermittelt, für die die Summe der Abweichungen der modellierten Impedanzen $\underline{Z}_{mod}$ als Modellausgangsgröße ($\underline{A}_{mod}$) zu den gemessenen Impedanzen $\underline{Z}_{mess}$ als Messgröße ($A_{mess}$) minimal sind.

In einer alternativen Ausführungsform kann in den Schritten d. - f. auch ein Spannungsverlauf U($\omega_i$) an einer Erregerspule eingeprägt werden, und aus dem sich einstellenden Stromverlauf $\underline{I}_{mess}(\omega_i)$ in der Messspule die Impedanzen $\underline{Z}_{mess}(\omega_i)$ bestimmt werden.

In einer weiteren bevorzugten Ausführungsform wird eine Spannung als Modellausgangsgröße $A_{mod}$ modelliert, die durch das Einprägen eines definierten Stromverlaufs induziert wird, d. h. die modellierte Größe $\underline{A}_{mod}$ ist eine Spannung $\underline{U}_{mod}$. Dabei sind die Schritte des Verfahrens wie folgt der Reihe nach:

    a. Bereitstellen eines mathematischen Models. Das Modell beschreibt die Spannungen $\underline{U}_{mod}(\omega, I)$ in der Messspule eines Messkreises mit einer Probe 4 eines Eisenschwamms in Abhängigkeit des Gehalts an metallischem Eisen (Fe$_{met}$), Eisenoxiden (Fe$_2$O$_3$, Fe$_3$O$_4$ bzw. FeO) und Eisenkarbid (Fe$_3$C) des Eisenschwamms bzw. der Probe 4 davon. Für diese Beschreibung weist das mathematische Modell 1 einerseits eine Bestimmung der Impedanz in Abhängigkeit der Permeabilität ($\mu_{eff}$) und der elektrischen Leitfähigkeit ($\sigma_{eff}$) des Eisenschwamms oder einer Probe 4 davon auf. Andererseits weist das Modell eine Bestimmung der Permeabilität ($\mu_{eff}$) und für die elektrische Leitfähigkeit ($\sigma_{eff}$) in Abhängigkeit des Gehalts an metallischem Eisen ($Fe_{met}$), Eisenoxiden ($Fe_2O_3$, $Fe_3O_4$ bzw. FeO) und Eisenkarbid ($Fe_3C$) des Eisenschwamms, sowie des Gangartanteils und der Dichte auf, wozu eine Effektive-Medium-Approximation (EMA) zur Anwendung kommt.

b. Einsetzen der effektive Dichte ptot der Probe 4 und des Gangartanteils $p_g$ der Probe 4 in das mathematische Modell 1.

c. Einbringen einer Probe 4 des Eisenschwamms in den magnetischen Kreis der Messanordnung.

d. Einprägen eines ersten Stromverlaufs $\underline{I}(\omega_1)$ in die Erregerspule samt Messung/Erfassung eines ersten Spannungsverlaufs $\underline{U}_{mess}(\omega_1)$ als Messgröße ($\underline{A}_{mess}$) an der Messspule.

e. Einprägen eines zweiten Stromverlaufs $\underline{I}(\omega_2)$ in die Erregerspule samt Messung/Erfassung eines zweiten Spannungsverlaufs $\underline{U}_{mess}(\omega_2)$ als Messgröße ($\underline{A}_{mess}$) an der Messspule.

f. Optional: Einprägen weiterer Stromverläufe $\underline{I}(\omega_i)$ in die Erregerspule samt Messungen/Erfassung weiterer Spannungsverläufe $\underline{U}_{mess}(\omega_i)$ als Messgröße ($\underline{A}_{m\text{-}ess}$) an der Messspule.

g. Bestimmung der Anteile an metallischem Eisen ($Fe_{met}$) und/oder Eisenoxid ($Fe_2O_3$, $Fe_3O_4$ bzw. FeO) und/oder Eisenkarbid ($Fe_3C$) in der Probe 4 an Eisenschwamm durch Anwendung eines Least-Squares-Schätzverfahrens unter Verwendung des mathematischen Modells 1 und der Messdaten für die Spannungsverläufe $\underline{U}_{mess}(\omega_1)$, $\underline{U}_{mess}(\omega_2)$ und optional $\underline{U}_{mess}(\omega_i)$.

Dabei werden jene Anteile an metallischem Eisen ($Fe_{met}$) und/oder Eisenoxid ($Fe_2O_3$, $Fe_3O_4$ und FeO) und/oder Eisenkarbid ($Fe_3C$) in der Probe 4 ermittelt, für die die Summe der Abweichungen der modellierten Spannungen $\underline{U}_{mod}$ als Modellausgangsgröße ($\underline{A}_{mod}$) zu den gemessenen Spannungen $\underline{U}_{mess}$ als Messgröße ($\underline{A}_{mess}$) minimal sind.

Damit wird mit dem diese berücksichtigenden mathematischen Modell 1 mit approximierter Permeabilität $\mu_{eff}$ und elektrischer Leitfähigkeit $\sigma_{eff}$ für ein Impedanzmodell 3 und vorzugsweise unter Verwendung der bekannten Parameter effektive Dichte $\rho_{tot}$ und Gangartanteil $p_g$ eine besonders genaue Gehaltsbestimmung von metallischem Eisen ($Fe_{met}$), optional Eisenoxid (FeO) sowie optional Eisenkarbid ($Fe_3C$) des Eisenschwamms bzw. einer Probe 4 davon ermöglicht.

## Patentansprüche

1. Verfahren zur Gehaltsbestimmung zumindest von metallischem Eisen ($Fe_{met}$) in durch Direktreduktion aus Eisenerz hergestelltem Eisenschwamm oder einer Probe (4) davon, aufweisend folgende Verfahrensschritte:

   Erfassung mindestens einer, insbesondere elektrischen, Messgröße ($\underline{A}_{mess}$), die von mindestens einer elektromagnetischen Eigenschaft des Eisenschwamms oder einer Probe (4) davon abhängig ist, Bereitstellung eines mathematischen Modells (1), dessen Modellausgangsgröße ($\underline{A}_{mod}$) als Funktion des Gehalts zumindest von metallischem Eisen ($Fe_{met}$) des Eisenschwamms oder einer Probe (4) davon beschrieben wird, wobei dieses mathematische Modell (1) eine Effektive-Medium-Approximation (EMA) für die Permeabilität ($\mu_{eff}$) und für die elektrische Leitfähigkeit ($\sigma_{eff}$) des Eisenschwamms oder einer Probe (4) davon aufweist, Durchführung eines Schätzverfahrens zur Gehaltsbestimmung zumindest von metallischem Eisen ($Fe_{met}$) unter Verwendung der erfassten Messgröße ($\underline{A}_{mess}$) und des mathematischen Modells (1).

2. Verfahren zur Gehaltsbestimmung nach Anspruch 1, **dadurch gekennzeichnet, dass** in das mathematische Modell (1) ein Gangartanteil ($p_g$) des Eisenerzes oder Eisenschwamms oder einer Probe (4) davon als erster Eingangsparameter und/oder eine, insbesondere effektive, Dichte ($\rho_{tot}$) des Eisenschwamms oder einer Probe (4) davon als zweiter Eingangsparameter eingeht bzw. eingehen.

3. Verfahren zur Gehaltsbestimmung nach Anspruch 1 oder 2, **dadurch gekennzeichnet, dass** das mathematische Modell (1) ein elektromagnetisches Modell (EMM) ist, das insbesondere ein Impedanzmodell (3) umfasst.

4. Verfahren zur Gehaltsbestimmung nach Anspruch 3, **dadurch gekennzeichnet, dass** das Impedanzmodell (3) eine Modellierung zumindest einer elektrischen Spule (101) mit Eisenschwamm oder einer Probe (4) davon als Material des Kerns aufweist.

5. Verfahren zur Gehaltsbestimmung nach Anspruch 3 oder 4, **dadurch gekennzeichnet, dass** die Modellausgangsgröße ($\underline{A}_{mod}$) eine elektrisch Impedanz ($\underline{Z}_{mod}(\omega_i)$) ist.

6. Verfahren zur Gehaltsbestimmung nach einem der Ansprüche 3 oder 4, **dadurch gekennzeichnet, dass** die Mo-

dellausgangsgröße ($\underline{A}_{mod}$) eine elektrische Spannung ($\underline{U}_{mod}$ ($\omega_i$)) ist, die sich unter Berücksichtigung eines bekannten, insbesondere eingeprägten, Stroms ($\underline{I}$) an einer elektrischen Impedanz ($\underline{Z}_{mod}(\omega_i)$) des Impedanzmodells (3) bildet.

7. Verfahren zur Gehaltsbestimmung nach einem der Ansprüche 3 oder 4, **dadurch gekennzeichnet, dass** die Modellausgangsgröße ($\underline{A}_{mod}$) ein elektrischer Strom ($\underline{I}_{mod}(\omega_i)$) ist, der sich bei einem Anlegen einer bekannten, insbesondere eingeprägten, Spannung an einer elektrischen Impedanz ($\underline{Z}_{mod}(\omega_i)$) des Impedanzmodells (3) bildet.

8. Verfahren zur Gehaltsbestimmung nach einem der Ansprüche 1 bis 7, **dadurch gekennzeichnet, dass** durch das Schätzverfahren, insbesondere Least-Squares-Schätzverfahren, jener Gehalt oder jene Gehälter bestimmt werden, für welche die Abweichung der mindestens einen erfassten Messgröße ($\underline{A}_{mess}$), insbesondere der Summe der erfassten Messgrößen ($\underline{A}_{mess}$), zur Modellausgangsgröße ($\underline{A}_{mod}$) des mathematischen Modells (1) minimal ist.

9. Verfahren zur Gehaltsbestimmung nach einem der Ansprüche 1 bis 8, **dadurch gekennzeichnet, dass** als oder für die Messgröße ($\underline{A}_{mess}$) jene in einer Spule (101) induzierte elektrische Spannung ($\underline{U}_{mess}$) erfasst wird, die sich aus dem magnetischen Feld ergibt, das durch einen eingeprägten Strom ($\underline{I}$) erzeugt wird, wobei die Spule (101) Eisenschwamm oder eine Probe (4) davon als Material des Kerns aufweist.

10. Verfahren zur Gehaltsbestimmung nach einem der Ansprüche 1 bis 8, **dadurch gekennzeichnet, dass** als oder für die Messgröße ($\underline{A}_{mess}$) jener elektrische Strom ($\underline{I}_{mess}$) erfasst wird, der sich einstellt, wenn an einer Spule (101) eine eingeprägte Spannung (U) angelegt wird, wobei die Spule (101) Eisenschwamm oder eine Probe (4) davon als Material des Kerns aufweist.

11. Verfahren zur Gehaltsbestimmung nach einem der Ansprüche 1 bis 10, **dadurch gekennzeichnet, dass** erste Messdaten ($\underline{A}_{mess}(\omega_1)$) der Messgröße ($\underline{A}_{mess}$) unter Anlegen eines ersten magnetischen Felds an den Eisenschwamm oder einer Probe (4) davon und zweite Messdaten ($\underline{A}_{mess}$ ($\omega_2$)) der Messgröße ($\underline{A}_{mess}$) unter Anlegen eines zweiten magnetischen Felds an den Eisenschwamm oder einer Probe (4) davon erfasst werden, wobei sich die magnetischen Felder zeitlich ändern und zueinander unterschiedliche Frequenzen aufweisen.

12. Verfahren zur Gehaltsbestimmung nach einem der Ansprüche 1 bis 11, **dadurch gekennzeichnet, dass** die Modellausgangsgröße ($\underline{A}_{mod}$) als Funktion des Gehalts zumindest von metallischem Eisen ($Fe_{met}$) sowie von mindestens einem Eisenoxid ($Fe_2O_3$, $Fe_3O_4$ bzw. FeO) als auch von Eisenkarbid ($Fe_3C$) des Eisenschwamms oder einer Probe (4) davon beschrieben wird, und das Schätzverfahren zur Gehaltsbestimmung zumindest von metallischem Eisen ($Fe_{met}$), Eisenoxid ($Fe_2O_3$, $Fe_3O_4$ bzw. FeO) und Eisenkarbid ($Fe_3C$) unter Verwendung der erfassten Messgröße ($\underline{A}_{mess}$) und des mathematischen Modells (1) durchgeführt wird.

**Claims**

1. Method for determining the content of at least metallic iron ($Fe_{met}$) in sponge iron produced by direct reduction from iron ore or a sample (4) thereof, comprising the following method steps:

   acquisition of at least one, more particularly electrical, measurand ($\underline{A}_{mess}$) which is dependent on at least one electromagnetic property of the sponge iron or a sample (4) thereof,
   providing a mathematical model (1) whose model output variable ($\underline{A}_{mod}$) is described as a function of the content of at least metallic iron ($Fe_{met}$) of the sponge iron or a sample (4) thereof, wherein this mathematical model (1) has an effective medium approximation (EMA) for the permeability ($\mu_{eff}$) and for the electrical conductivity ($\sigma_{eff}$) of the sponge iron or a sample (4) thereof,
   carrying out an estimation method for determining the content of at least metallic iron ($Fe_{met}$) using the measurand ($\underline{A}_{mess}$) and the mathematical model (1).

2. Method for determining the content according to claim 1, **characterized in that** a gangue fraction ($p_g$) of the iron ore or sponge iron or of a sample (4) thereof enters into the mathematical model (1) as a first input parameter and/or a, more particularly effective, density (ptot) of the sponge iron or of a sample (4) thereof as a second input parameter.

3. Method for determining the content according to claim 1 or 2, **characterized in that** the mathematical model (1) is an electromagnetic model (EMM), which more particularly comprises an impedance model (3).

**EP 4 180 801 B1**

4. Method for determining the content according to claim 3, **characterized in that** the impedance model (3) comprises a modeling of at least one electric coil (101) with sponge iron or a sample (4) thereof as the material of the core.

5. Method for determining the content according to claim 3 or 4, **characterized in that** the model output variable ($\underline{A}_{mod}$) is an electrical impedance ($\underline{Z}_{mod}(\omega_i)$).

6. Method for determining the content according to one of claims 3 or 4, **characterized in that** the model output variable ($\underline{A}_{mod}$) is an electrical voltage ($\underline{U}_{mod}(\omega_i)$) which is formed by considering a known, more particularly impressed, current (I) at an electrical impedance ($\underline{Z}_{mod}(\omega_i)$) of the impedance model (3).

7. Method for determining the content according to one of claims 3 or 4, **characterized in that** the model output variable ($\underline{A}_{mod}$) is an electric current ($\underline{I}_{mod}(\omega_i)$) which is formed when a known, more particularly impressed, voltage is applied to an electric impedance ($\underline{Z}_{mod}(\omega_i)$) of the impedance model (3).

8. Method for determining the content according to one of claims 1 to 7, **characterized in that** the estimation method, more particularly the least squares estimation method, is used to determine that content or those contents for which the deviation of the at least one recorded measurand ($\underline{A}_{mess}$), more particularly the sum of the recorded measurands ($\underline{A}_{mess}$), from the model output variable ($\underline{A}_{mod}$) of the mathematical model (1) is minimal.

9. Method for determining the content according to one of claims 1 to 8, **characterized in that** such electric voltage ($\underline{U}_{mess}$) is recorded as or for the measurand ($\underline{A}_{mess}$) which is induced in a coil (101) and which results from the magnetic field generated by an impressed current (I), wherein the coil (101) has sponge iron or a sample (4) thereof as material of the core.

10. Method for determining the content according to one of claims 1 to 8, **characterized in that** such electric current ($\underline{I}_{mess}$) which occurs when an impressed voltage (U) is applied to a coil (101) is recorded as or for the measurand ($\underline{A}_{mess}$), wherein the coil (101) has sponge iron or a sample (4) thereof as the material of the core.

11. Method for determining the content according to one of claims 1 to 10, **characterized in that** first measurement data ($\underline{A}_{mess}(\omega_1)$) of the measurand ($\underline{A}_{mess}$) are recorded with the application of a first magnetic field to the sponge iron or a sample (4) thereof and second measurement data ($\underline{A}_{mess}(\omega_2)$) of the measurand ($\underline{A}_{mess}$) are recorded with the application of a second magnetic field to the sponge iron or a sample (4) thereof, wherein the magnetic fields change over time and have different frequencies from one another.

12. Method for determining the content according to one of claims 1 to 11, **characterized in that** the model output variable ($\underline{A}_{mod}$) is determined as a function of the content of at least metallic iron ($Fe_{met}$) as well as of at least one iron oxide ($Fe_2O_3$, $Fe_3O_4$ or FeO) as well as of iron carbide ($Fe_3C$) of the sponge iron or a sample (4) thereof, and the estimation method for determining the content of at least metallic iron ($Fe_{met}$), iron oxide ($Fe_2O_3$, $Fe_3O_4$ or FeO) and iron carbide ($Fe_3C$) is carried out using the recorded measurand ($\underline{A}_{mess}$) and the mathematical model (1).

**Revendications**

1. Procédé de détermination de la teneur au moins en fer métallique ($Fe_{met}$) dans une éponge de fer produite par réduction directe à partir de minerai de fer ou dans un échantillon (4) de celle-ci, présentant les étapes de procédé suivantes :

   acquisition d'au moins une grandeur de mesure ($\underline{A}_{mess}$), en particulier électrique, qui dépend d'au moins une propriété électromagnétique de l'éponge de fer ou d'un échantillon (4) de celle-ci,
   mise à disposition d'un modèle mathématique (1) dont la grandeur de sortie de modèle ($\underline{A}_{mod}$) est décrite en fonction de la teneur au moins en fer métallique ($Fe_{met}$) de l'éponge de fer ou d'un échantillon (4) de celle-ci, ce modèle mathématique (1) présentant une approximation du milieu effectif (EMA) pour la perméabilité ($\mu_{eff}$) et pour la conductivité électrique ($\sigma_{eff}$) de l'éponge de fer ou d'un échantillon (4) de celle-ci,
   mise en oeuvre d'un procédé d'estimation pour la détermination de la teneur au moins en fer métallique ($Fe_{met}$) en utilisant la grandeur de mesure acquise ($\underline{A}_{mess}$) et le modèle mathématique (1).

2. Procédé de détermination de la teneur selon la revendication 1, **caractérisé en ce que** dans le modèle mathématique (1), une proportion de gangue ($p_g$) du minerai de fer ou de l'éponge de fer ou d'un échantillon (4) de celle-ci est ou

9

sont introduits comme premier paramètre d'entrée et/ou une densité ($\rho_{tot}$), en particulier effective, de l'éponge de fer ou d'un échantillon (4) de celle-ci comme deuxième paramètre d'entrée.

3. Procédé de détermination de la teneur selon la revendication 1 ou 2, **caractérisé en ce que** le modèle mathématique (1) est un modèle électromagnétique (EMM), qui comprend en particulier un modèle d'impédance (3).

4. Procédé de détermination de la teneur selon la revendication 3, **caractérisé en ce que** le modèle d'impédance (3) présente une modélisation d'au moins une bobine électrique (101) avec de l'éponge de fer ou un échantillon (4) de celle-ci comme matériau du noyau.

5. Procédé de détermination de la teneur selon la revendication 3 ou 4, **caractérisé en ce que** la grandeur de sortie de modèle ($\underline{A}_{mod}$) est une impédance électrique ($\underline{Z}_{mod}(\omega_i)$).

6. Procédé de détermination de la teneur selon l'une des revendications 3 ou 4, **caractérisé en ce que** la grandeur de sortie de modèle ($\underline{A}_{mod}$) est une tension électrique ($\underline{U}_{mod}(\omega_i)$) qui se forme en tenant compte d'un courant connu, en particulier imposé ($\underline{I}$), à une impédance électrique ($\underline{Z}_{mod}(\omega_i)$) du modèle d'impédance (3).

7. Procédé de détermination de la teneur selon l'une des revendications 3 ou 4, **caractérisé en ce que** la grandeur de sortie de modèle ($\underline{A}_{mod}$) est un courant électrique ($\underline{I}_{mod}(\omega_i)$) qui se forme lors de l'application d'une tension connue, en particulier imposée, à une impédance électrique ($\underline{Z}_{mod}(\omega_i)$) du modèle d'impédance (3).

8. Procédé de détermination de la teneur selon l'une des revendications 1 à 7, **caractérisé en ce que** le procédé d'estimation, en particulier un procédé d'estimation par moindres carrés, permet de déterminer la teneur ou les teneurs pour lesquelles l'écart de ladite au moins une grandeur de mesure acquise ($\underline{A}_{mess}$), en particulier la somme des grandeurs de mesure acquises ($\underline{A}_{mess}$), par rapport à la grandeur de sortie de modèle ($\underline{A}_{mod}$) du modèle mathématique (1) est minimal.

9. Procédé de détermination de la teneur selon l'une des revendications 1 à 8, **caractérisé en ce que** l'on acquiert comme ou pour la grandeur de mesure ($\underline{A}_{mess}$) la tension électrique ($\underline{U}_{mess}$) induite dans une bobine (101), qui résulte du champ magnétique qui est généré par un courant imposé ($\underline{I}$), la bobine (101) présentant de l'éponge de fer ou un échantillon (4) de celle-ci comme matériau du noyau.

10. Procédé de détermination de la teneur selon l'une des revendications 1 à 8, **caractérisé en ce que** l'on acquiert comme ou pour la grandeur de mesure ($A_{mess}$) le courant électrique ($\underline{I}_{mess}$) qui s'établit lorsqu'une tension imposée ($\underline{U}$) est appliquée à une bobine (101), la bobine (101) présentant de l'éponge de fer ou un échantillon (4) de celle-ci comme matériau du noyau.

11. Procédé de détermination de la teneur selon l'une des revendications 1 à 10, **caractérisé en ce que** des premières données de mesure ($\underline{A}_{mess}(\omega_1)$) de la grandeur de mesure ($\underline{A}_{mess}$) sont acquises en appliquant un premier champ magnétique à l'éponge de fer ou à un échantillon (4) de celle-ci et des deuxièmes données de mesure ($\underline{A}_{mess}(\omega_2)$) de la grandeur de mesure ($\underline{A}_{mess}$) sont acquises en appliquant un deuxième champ magnétique à l'éponge de fer ou à un échantillon (4) de celle-ci, les champs magnétiques variant dans le temps et présentant des fréquences différentes l'une de l'autre.

12. Procédé de détermination de la teneur selon l'une des revendications 1 à 11, **caractérisé en ce que** la grandeur de sortie de modèle ($\underline{A}_{mod}$) est déterminée en fonction de la teneur au moins en fer métallique ($Fe_{met}$) ainsi qu'en au moins un oxyde de fer ($Fe_2O_3$, $Fe_3O_4$ ou $FeO$) ainsi que de carbure de fer ($Fe_3C$) de l'éponge de fer ou d'un échantillon (4) de celle-ci, et le procédé d'estimation pour la détermination de la teneur au moins en fer métallique ($Fe_{met}$), oxyde de fer ($Fe_2O_3$, $Fe_3O_4$ ou $FeO$) et carbure de fer ($Fe_3C$) est mis en oeuvre en utilisant la grandeur de mesure acquise ($\underline{A}_{mess}$) et le modèle mathématique (1).

FIG.1

EP 4 180 801 B1

*FIG.2*

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 3017001 A1

### In der Beschreibung aufgeführte Nicht-Patentliteratur

- **H. LIBBY.** Introduction to Electromagnetic Nondestructive Test Methods. Wiley, 1971
- **J. C. MAXWELL-GARNETT.** Colours in metal glasses and in metal films. *Trans. Royal Soc. London,* 1904, vol. 203, 385-420
- **A. H. SIHVOLA ; I. V. LINDELL.** Effective permeability of mixtures. *Prog Electromagn. Res.,* 1992, vol. 06, 153-180